# FASCICULE DE BREVET EUROPEEN

(11) **EP 1 390 007 B1**
(45) Date de publication et mention de la délivrance du brevet: **08.10.2008**
(21) Numéro de dépôt: 02738218.3
(22) Date de dépôt: 07.05.2002
(51) Int. Cl.: A61K 8/19, A61K 8/26, A61K 8/60, A61K 8/27, A61K 9/10, A61K 33/30, A61Q 19/00

(54) **COMPOSITION COSMETIQUE A BASE DE SUCRALFATE ET DE SULFATES DE CUIVRE ET DE ZINC**
KOSMETISCHE ZUSAMMENSETZUNG AUF BASIS VON ZINK- UND KUPFERSULFATEN UND SUCRALPHAT
COSMETIC COMPOSITION BASED ON ZINC AND COPPER SULPHATES AND SUCRALPHATE

(30) Priorité: 11.05.2001 FR 0106242
(43) Date de publication de la demande: 25.02.2004
(73) Titulaire: Pierre Fabre Dermo-Cosmétique, 92100 Boulogne-Billancourt (FR)
(72) Inventeur: FABRE, Pierre, F-81106 Castres (FR); DUSSERT, Anne-Sophie, F-31500 Toulouse (FR); JEANJEAN, Michel, F-31329 Castanet Tolosan (FR)
(74) Mandataire: Ahner, Francis
(86) Numéro de dépôt international: PCT/FR2002/001563
(87) Numéro de publication internationale: WO 2002/092045

(56) Documents cités:
- WO-A-94/27579
- WO-A-99/43333
- FR-A- 2 646 604
- US-A- 4 945 084

## Description

La présente invention concerne des formulations cosmétiques contenant du sucralfate en association avec du sulfate de cuivre et du sulfate de zinc utilisés comme régénérant tissulaire, cicatrisant et anti-inflammatoire.

Le sucralfate est du saccharose sulfate d'aluminium basique, il est utilisé comme médicament dans le traitement des ulcères gastriques et duodénaux sous les noms de marque ULCAR^{®} et KEAL^{®}.

Absorbé à la dose de 0,5 à 2 g jour sous une forme sèche type comprimé ou granulés à croquer, le sucralfate agit sur les tractus digestif en recouvrant la muqueuse de l'estomac et du duodénum d'un gel protecteur.

La formation de ce gel est consécutive à la réaction qui se produit entre le sucralfate et l'acide chlorhydrique du milieu gastrique et duodénal et en raison du tropisme électromagnétique qu'il présente pour les molécules protéiniques, chargés positivement, il forme avec elles un complexe qui isole et protège l'ulcère gastrique.

Par ailleurs, le sucralfate inhibe l'activité protéolytique de la pepsine. Ainsi, il permet et favorise la cicatrisation naturelle de l'ulcère.

FR-A-2646604 décrit des formulations de sucralfate aux propriétés anti-inflammatoires et cicatrisantes destinées au traitement de plaies ou autres inflammations ulcéreuses externe. Cependant, afin d'obtenir des compositions de sucralfate stables, la composition doit être acidifiée à un pH proche de 4,5.

La présente invention concerne l'utilisation cosmétique donc par voie topique de formulations contenant du sucralfate en association avec du sulfate de cuivre et du sulfate de zinc comme régénérant tissulaire, cicatrisant et apaisant.

Selon une caractéristique particulière de la présente invention, la composition cosmétique présente une teneur en sucralfate comprise 0,01 % et 5% en poids, et de préférence environ 1% en poids.

Selon une autre caractéristique de la présente invention, la composition cosmétique comprend de 0,02 % à 2% en poids de sulfates, et de préférence entre 0,3 % et 2% en poids.

Selon une autre caractéristique de la présente invention, la composition comprend un rapport en poids de sucrafalte aux sulfates compris entre 0, 5 et 20 et de préférence entre 0,5 et 1.

Selon une autre caractéristique de la présente invention, la composition cosmétique contient un rapport en poids de sulfate de cuivre au sulfate de zinc compris entre 1 et 3.

Les exemples de formulations donnés ci-après sont destinés à illustrer l'invention et ne sont cités qu'à titre non limitatif.

### Exemple 1 : Crème eau dans huile

| | |
|---|---|
| eau thermale d'Avène q.s. | 100 g |
| sucralfate micronisé | 1 g |
| sulfate de cuivre | 0,2 g |
| sulfate de zinc | 0,1 g |
| oxyde de zinc | 4 g |
| glycérol | 5 g |
| Hostacérine WO | |
| (polyglycéryl-2 sesquiisostéarate | |
| + cire d'abeille + huile minérale | |
| + stéarate de magnésium et d'aluminium) | 3,7 g |
| Crémiol HF52 (huile végétale hydrogénée) | 5 g |
| Paraffine liquide | 8 g |
| triglycéride caprylique -caprique | 19 g |
| Elfaros ST 37 | 1,2 g |
| (PEG 22 dodécylglycolcopolymère) | |
| propylène glycol | 3 g |
| sulfate de magnésium | 0,1 g |

### Exemple 2 : Emulsion 1 (huile dans eau)

| | |
|---|---|
| Triglycéride caprylique - caprique | 7 g |
| Huile de carthame | 7 g |
| Glycéryl stéarate + alcool stéarilique | |
| + ceteth 20 + stéareth 25 | 6,5 g |
| Beurre de Karité | 3 g |
| Diméthicone | 2 g |
| Sodium Carbomer | 0,35 g |
| Sucralfate | 0,01 g |
| Sulfate de cuivre | 0,01 g |
| Sulfate de zinc | 0,01 g |
| Eau déminéralisée Q.S.P. | 100 mg |

### Exemple 3 : Emulsion 2 (H/E)

| | |
|---|---|
| Huile de paraffine | 10 g |
| Triglycéride caprylique - caprique | 7 g |
| Cyclométhicone | 3 g |
| Sucrose stéarate | 2 g |
| Sucrose distéarate | 2 g |
| Carbomer | 0,4 g |
| Cakilé | 2 g |
| Triéthanolamine Q.S.P. | PH 7 |
| Sucralfate | 5 g |
| Sulfate de zinc | 0,2 g |
| Sulfate de cuivre | 0,2 g |
| Eau déminéralisée Q.S.P. | 100 g |

### Exemple 4 : Emulsion 3 (H/E)

| | |
|---|---|
| Cyclométhicone | 10 g |
| Cetyl diméthicone copolyol + polyglycéryl | |
| 4-isostéarate + hexylaurate | 3 g |
| Huile de carthame | 4 g |
| Glycérine | 10 g |
| PEG 12 | 10 g |
| Silicate double d'aluminium et de | |
| Magnésium | 1,5 g |
| Sucralfate | 3 g |
| Sulfate de cuivre | 0,3 g |
| Sulfate de zinc | 0,1 g |
| eau déminéralisée Q.S.P. | 100 g |

### Exemple 5 : Emulsion 4 (H/E)

| | |
|---|---|
| Sépigel 305 | 3,5 g |
| Cyclométhécone | 6 g |
| Propylène glycol | 5 g |
| Gomme xanthane | 0,2 g |
| Triéthanolamine Q.S.P. | PH 6,5 |
| Sucralfate | 0,5 g |
| Sulfate de cuivre | 0,1 g |
| Sulfate de zinc | 0,1 g |
| eau déminéralisée Q.S.P. | 100 g |

### Exemple 6 : Pommade

| | |
|---|---|
| Vaseline | 10 g |
| Paraffine liquide | 8 g |
| Cire d'abeille | 4 g |
| Palmitate d'isopropyle | 11 g |
| Squalane | 5 g |
| Ozokerite | 9 g |
| Lanoline hydrogénée | 10 g |
| Beurre de karité | 2 g |
| Sucralfate | 1 g |
| Sulfate de zinc | 0,1 g |
| Sulfate de cuivre | 0,1 g |
| Huile de ricin Q.S.P. | 100 g |

### Exemple 7 : Vaporisateur à pompe

| | |
|---|---|
| Silicates d'aluminium et de magnésium | 5 g |
| Sucralfate | 1 g |
| Sulfate de cuivre | 1 g |
| Sulfate de zinc | 1 g |
| Eau d'Avène Q.S.P. | 100 g |

### Exemple 8 : Spray poudre aérosol

| | |
|---|---|
| Sucralfate micronisé | 2 g |
| Sulfate de cuivre | 0,2 g |
| Sulfate de zinc | 0,2 g |
| Oxyde de zinc | 0,3 g |
| Décaméthylcyclosiloxane | 10 g |
| Quaternium 18 Hectorite | 1,2 g |
| Mélange propulseur (isobutane, propane, | |
| N-butane) QSP (100 ml) | |

### Evaluation dermocosmétique

Le but de cette étude a été d'évaluer les propriétés régénérante, cicatrisante et apaisante de la crème de l'exemple 1.

Le modèle expérimental retenu a été celui de la bulle de succion. Il s'agit d'une technique classique, généralement utilisée pour étudier comparativement une surface de peau non traitée, l'effet d'un produit sur la vitesse et la qualité de ré-épidermisation d'une surface parfaitement délimitée de peau dont on a préalablement découpée la couche épidermique au-dessus de la fonction dermo-épidermique.

### Méthodologie

L'essai a été réalisé sur 6 volontaires. La zone expérimentale choisie est la face interne avant-bras (zone peu exposée aux rayonnements ultraviolets et à d'éventuelles agressions mécaniques externes) sur laquelle six bulles de succion à contours parfaitement délimités ont été réalisées.

Après retrait de l'épiderme décollé, cinq bulles ont reçu la crème de l'exemple 1 et/ou les excipients de cette crème (c'est-à-dire sans le sucralfate, le sulfate de cuivre et le sulfate de zinc). La 6ème bulle a été considérée comme témoin non traité. L'application du ou des excipients a été réalisée quotidiennement par un dermatologue pendant 14 jours consécutifs.

Chaque produit a été prélevé à l'aide d'une seringue (sans aiguille) stérile à usage unique, puis déposé sur toute la surface de la bulle de succion afin de former une couche uniforme d'environ 1 mm d'épaisseur.

Chaque application a été précédée d'un nettoyage des bulles à traiter à l'aide de compresses stériles imprégnées de sérum physiologie stérile, par léger tamponnage vertical. Chaque bulle a été ensuite recouverte d'une compresse stérile fixée au moyen d'un pansement adhésif. Les pansements ont été laissés in situ jusqu'à l'observation clinique suivante.

Les propriétés régénérantes ont été appréciées par une méthode quanti-qualitative permettant de mesurer la vitesse et la qualité de l'épidermisation sur une période de 14 jours. La vitesse d'épidermisation a été calculée (après mesure des surfaces lésées par analyse d'image) selon la formule ST-S0/T avec ST = surface lésée au temps T et S0 = surface lésée au temps T0. T étant le temps où une première épidermisation totale est obtenue chez un volontaire.

La qualité de l'épidermisation a été appréciée par la comparaison des critères cliniques relatifs à la qualité de peau obtenue lors des contrôles à J14 et J1 (avant la formation des bulles de succion).

Les critères d'évaluation ont été les suivants :
- intensité de l'érythème (1 érythème léger, 2 érythème modéré, 3 érythème sévère)
- finesse de la peau (0 très fine, 1 fine, 2 épaisse)
- souplesse de la peau (0 pas souple, 1 souple, 2 très souple)
- normalité de la régénération épidermique (oui-non) sachant qu'une cicatrice anormale peut se caractériser par une hyper-hypotrophie ou un hyper-hypopigmentation

### Résultats

### • Vitesse d'épidermisation

La crème de l'exemple 1 a une vitesse d'épidermisation moyenne (V1 = 11,72 mm2/j) 2 fois plus rapide que celle observée sur une bulle non traitée et une ré-épidermisation complète de 100 % des bulles à J4.

L'excipient de cette crème a une vitesse d'épidermisation moyenne (V2 - 8 mm2/j) 1,5 fois plus rapide que celle observée sur bulles témoins non traitées et une ré-épidermisation de 70 % de bulles à J4.

### • Evaluation de la qualité de l'épidermisation entre J1 et J4

### Intensité de l'érythème

Crème de l'exemple 1 < excipient de l'exemple 1 < témoin

### Finesse de la peau

Crème de l'exemple 1> excipient de l'exemple 1 > témoin

### Souplesse de la peau

Crème de l'exemple 1> excipient de l'exemple 1 > témoin

### Epidérmisation normale (à J14)

Crème de l'exemple 1> excipient de l'exemple 1 > témoin

La présente invention s'étend donc également à l'utilisation d'une association de sucralfate et de sulfates de cuivre et de zinc dans les quantités et proportions déjà énoncées précédemment, pour la fabrication d'une composition dermo-cosmétique destinée à un traitement régénérant, cicatrisant et/ou anti-inflammatoire de la peau.

## Revendications

1. Composition cosmétique comprenant une association de sucralfate et d'un mélange de sulfates de cuivre et de zinc, dans un excipient permettant une application topique sur la peau.

2. Composition cosmétique selon la revendication 1, **caractérisée en ce qu'**elle contient de 0,01 % à 5 % en poids et de préférence environ 1% en poids de sucralfate.

3. Composition cosmétique selon l'une des revendications 1 et 2, **caractérisée en ce qu'**elle contient de 0,02 % à 2% en poids et de préférence de 0,3 % à 2% en poids d'un mélange de sulfates de cuivre et de zinc.

4. Composition cosmétique selon l'une des revendications 1 à 3, **caractérisée en ce que** le rapport en poids de sucralfate au mélange de sulfates de cuivre et de zinc est compris entre 0,5 et 20.

5. Composition cosmétique selon la revendication 4, **caractérisée en ce que** le rapport en poids de sucralfate au mélange de sulfates de cuivre et de zinc est compris entre 0,5 et 1.

6. Composition cosmétique selon l'une des revendications 1 à 5, **caractérisée en ce que** le rapport en poids de sulfate de cuivre au sulfate de zinc est compris entre 1 et 3.

7. Utilisation d'une association de sucralfate et d'un mélange de sulfates de cuivre et de zinc, en particulier dans les proportions indiquées aux revendications 2 à 6, pour la fabrication d'une composition dermo-cosmétique destinée à un traitement régénérant, cicatrisant et/ou anti-inflammatoire de la peau.

## Claims

1. A cosmetic composition comprising an association of sucralphate and of a mixture of copper and zinc sulphates, in an excipient allowing it to be topically applied on the skin.

2. The cosmetic composition according to claim 1, **characterized in that** it contains from 0.01% to 5% by weight and preferably about 1% by weight of sucralphate.

3. The cosmetic composition according to any of claims 1 and 2, **characterized in that** it contains from 0.02% to 2% by weight and preferably from 0.3% to 2% by weight of a mixture of copper and zinc sulphates.

4. The cosmetic composition according to any of claims 1 to 3, **characterized in that** the weight ratio of sucralphate to the mixture of copper and zinc sulphates is comprised between 0.5 and 20.

5. The cosmetic composition according to claim 4, **characterized in that** the weight ratio of sucralphate to the mixture of copper and zinc sulphates is comprised between 0.5 and 1.

6. The cosmetic composition according to any of claims 1 to 5, **characterized in that** the weight ratio of copper sulphate to zinc sulphate is comprised between 1 and 3.

7. The use of an association of sucralphate and a mixture of copper and zinc sulphates, in particular in the proportions indicated in claims 2 to 6, for making a dermocosmetic composition intended for a regenerating, healing and/or anti-inflammatory treatment of the skin.

## Patentansprüche

1. Kosmetische Zusammensetzung, die eine Assoziation von Sucralphat und eines Gemischs von Zink- und Kupfersulfaten in einem Exzipient, der eine topische Anwendung auf der Haut erlaubt, umfasst.

2. Kosmetische Zusammensetzung nach Anspruch 1, **dadurch gekennzeichnet, dass** sie 0,01 bis 5 Gew.-% und vorzugsweise zirka 1 Gew.-% Sucralphat enthält.

3. Kosmetische Zusammensetzung nach einem der Ansprüche 1 und 2, **dadurch gekennzeichnet, dass** sie 0,02 bis 2 Gew.-% und vorzugsweise 0,3 bis 2 Gew.-% eines Gemischs von Zink- und Kupfersulfaten enthält.

4. Kosmetische Zusammensetzung nach einem der Ansprüche 1 bis 3, **dadurch gekennzeichnet, dass** das Gewichtsverhältnis von Sucralphat zu dem Gemisch von Zink- und Kupfersulfaten zwischen 0,5 und 20 inklusive ist.

5. Kosmetische Zusammensetzung nach Anspruch 4, **dadurch gekennzeichnet, dass** das Gewichtsverhältnis von Sucralphat zu dem Gemisch von Zink- und Kupfersulfaten zwischen 0,5 und 1 inklusive ist.

6. Kosmetische Zusammensetzung nach einem der Ansprüche 1 bis 5, **dadurch gekennzeichnet, dass** das Gewichtsverhältnis von Kupfersulfat zu Zinksulfat zwischen 1 und 3 inklusive ist.

7. Verwendung einer Assoziation von Sucralphat und eines Gemischs von Zink- und Kupfersulfaten in den insbesondere in den Ansprüchen 2 bis 6 angegebenen Verhältnissen, zur Herstellung einer dermokosmetischen Zusammensetzung, die für eine regenerierende, heilende und/oder entzündungshemmende Behandlung der Haut bestimmt ist.
